# FASCICULE DE BREVET EUROPEEN

(11) **EP 0 613 354 B1**
(45) Date de publication et mention de la délivrance du brevet: **24.09.1997**
(21) Numéro de dépôt: 93900244.0
(22) Date de dépôt: 12.11.1992
(51) Int. Cl.: A61F 5/04, A61F 15/00

(54) **APPAREIL ORTHOPEDIQUE D'AIDE A LA CONFECTION D'IMMOBILISATION OU DE PANSEMENT DU MEMBRE INFERIEUR**
ORTHOPÄDISCHE VORRICHTUNG ZUM ANLEGEN EINES VERBANDES ODER ZUM UNBEWEGLICHMACHEN DER UNTEREN GLIEDMASSEN
ORTHOPEDIC APPARATUS FOR MAKING A DRESSING OR IMMOBILIZING LOWER MEMBERS

(30) Priorité: 19.11.1991 FR 9114523
(43) Date de publication de la demande: 07.09.1994
(73) Titulaire: PECHEUR, Antoine, 74220 La Clusaz (FR)
(72) Inventeur: PECHEUR, Antoine, 74220 La Clusaz (FR)
(74) Mandataire: Gasquet, Denis
(86) Numéro de dépôt international: FR9201051
(87) Numéro de publication internationale: WO9309735

(56) Documents cités:
- WO-A-91/09577
- DE-A- 2 163 561
- FR-A- 829 435
- GB-A- 9 537
- US-A- 2 362 866
- US-A- 2 486 687

## Description

### DOMAINE DE L'INVENTION

La présente invention concerne les appareils orthopédiques permettant la confection d'une immobilisation ou d'un pansement sur le membre inférieur du corps humain.

La réalisation d'une immobilisation de membre inférieur du corps humain, en plâtre ou en résine, nécessite de passer plusieurs fois une bande plâtre ou de résine autour du membre, tout en maintenant les articulations du membre dans une orientation fixe pendant la confection de l'immobilisation et pendant la prise ultérieure du plâtre ou de la résine.

Selon les techniques traditionnelles encore fréquemment utilisées, le médecin qui confectionne une immobilisation en plâtre ou en résine, se fait aider par un assistant qui maintient le membre dans la position appropriée et pendant la durée appropriée. Un tel maintien s'avère toutefois insuffisant, et conduit le plus souvent à une déformation du plâtre ou de la résine, pendant la prise du matériau, pouvant produire des points de compression du membre, douloureux et éventuellement dangereux.

Pour faciliter le maintien du membre, et en particulier pour assurer un appui du pied, le document WO-A-91/09577 propose un dispositif comprenant une planchette conformée et soutenue par des moyens support en une position inclinée pour supporter vers le haut, la plante de pied du membre inférieur à immobiliser. Dans ce document, la planchette est tenue dans sa position inclinée par une succession de tiges support dont une extrémité se fixe à une table support, et dont l'autre extrémité se fixe perpendiculairement à la planchette pour passer entre deux orteils du pied à tenir.

Un tel dispositif support nécessite de prévoir une forme particulière de table, susceptible de recevoir et de maintenir l'extrémité des tiges de liaison avec la planchette. D'autre part, la liaison entre la table et la planchette, assurée par les tiges, est relativement flexible, et conduit à des mouvements possibles de la planchette pendant le maintien du pied. De tels mouvements risquent de produire des mouvements des articulations elles-mêmes pendant la prise du plâtre ou de la résine, provoquant des déformations de l'immobilisation. Le maintien n'est ainsi pas suffisant.

Le document GB-A-9 537, qui détermine le préambule de la revendication 1, décrit un support de jambe destiné à supporter la partie inférieure du membre inférieur, c'est-à-dire la partie située entre le genou et la plante du pied. Ce support comprend une plaque inférieure à laquelle se raccorde une planchette articulée destinée à venir en appui contre la plante du pied. Un tel dispositif peut convenir pour réaliser une attelle destinée à maintenir la rectitude du membre inférieur et à constituer elle-même l'immobilisation. Toutefois, ce dispositif n'est pas adapté pour la réalisation d'une immobilisation en plâtre ou en résine devant ensuite permettre la marche du patient. En effets le médecin n'a pas accès à la face inférieure de la jambe qui se trouve posée sur le support, et ne peut donc pas passer les bandes de plâtre ou de plastique pour la confection de l'immobilisation. En outre, le dispositif ne permet pas le maintien du membre inférieur complet et en particulier le positionnement de la cuisse et de l'articulation du genou selon une angulation appropriée.

Le document US-A-2 362 866 décrit une attelle universelle pouvant maintenir le dos et les deux membres inférieurs d'un patient selon des angulations variables. Un tel dispositif réalise par lui-même l'immobilisation, mais n'est pas adapté pour la réalisation d'un immobilisation en plâtre ou en résine car le médecin n'a pas accès à la partie du corps du patient en appui sur le dispositif.

Les documents DE-A-21 63 561, FR-A- 829 435 et US-A-2 486 687 décrivent d'autres modes de réalisation d'attelles pour l'immobilisation d'un membre. Aucun de ces dispositifs ne permet de réaliser de façon simple, une immobilisation de membre inférieur en plâtre ou en résine.

### RESUME DE L'INVENTION

L'objet de la présente invention est de concevoir une nouvelle structure d'appareil orthopédique permettant d'assurer le maintien du membre inférieur du corps humain pendant la confection d'une immobilisation ou d'un pansement, le maintien présentant un caractère suffisamment rigide et non déformable pour éviter toute déformation sensible de l'immobilisation pendant la prise du plâtre ou de la résine, le maintien étant assuré par des moyens particulièrement simples et peu onéreux pouvant s'adapter sur tout type de table support, sans l'utilisation d'outils particuliers, le positionnement de l'appareil lui-même étant particulièrement rapide. C'est en effet, le propre poids du membre inférieur qui verrouille en position.

La présente invention a également pour avantage de proposer un nouvel appareil de maintien qui, dans une position de rangement, présente un encombrement particulièrement réduit et peut être aisément manipulé, tous les éléments de l'appareil étant solidaires les uns des autres.

En outre, l'appareil selon l'invention convient, sans aucune modification, pour l'immobilisation d'un membre droit ou d'un membre gauche, et quelle que soit la taille du patient.

L'appareil peut en outre servir au maintien soit du membre inférieur complet, soit de la jambe. Par ailleurs, l'appareil permet au patient de maintenir sa jambe dans la bonne position sans avoir à fournir d'efforts, et donc sans avoir à contracter ses muscles. Ces derniers étant parfaitement relâchés, une parfaite réalisation du pansement est possible. La position du membre inférieur étant verrouillée par le propre poids de celui-ci.

Pour cela, l'appareil selon l'invention comprend une planchette conformée et soutenue par des moyens support, en une position inclinée pour supporter vers le haut et vers l'arrière, la plante de pied d'un membre inférieur; la planchette comprend une arête inférieure se raccordant mécaniquement à une planche support, au voisinage d'une première extrémité de ladite planche support; la partie de planche support s'étendant depuis l'arête de raccordement de planchette jusqu'à sa seconde extrémité forme, avec la planchette, un angle obtus tel que la planchette est dans ladite position inclinée appropriée lorsque la planche est horizontale; des moyens de maintien tiennent la planchette selon l'angle obtus fixe par rapport à ladite planche; la partie de planche support, s'étendant depuis l'arête de raccordement de planchette jusqu'à sa seconde extrémité, a une longueur supérieure à 80 centimètres environ, de sorte qu'une portion au moins de la planche se trouve en appui sous le dos du patient lorsque le pied du patient repose sur la planchette.

Par ailleurs, la planche comprend une partie large, conformée pour recevoir le bassin du patient en décubitus dorsal. La planche peut avantageusement comprendre une partie intermédiaire de moindre largeur.

### BREVE DESCRIPTION DE DESSINS

D'autres objets, caractéristiques et avantages de la présente invention ressortiront de la description qui va suivre d'un mode de réalisation particulier, faite en relation avec les figures jointes, parmi lesquelles :
- la figure 1 est une vue en perspective d'un appareil orthopédique selon la présente invention;
- la figure 2 illustre, en vue de côté, la position habituelle d'un membre inférieur lors d'une confection d'immobilisation à l'aide de l'appareil selon la présente invention;
- la figure 3 représente, en vue de côté, un autre mode d'utilisation de l'appareil selon l'invention, pour la confection d'une immobilisation de la jambe.

### DESCRIPTION DES MODES DE REALISATION PREFERES

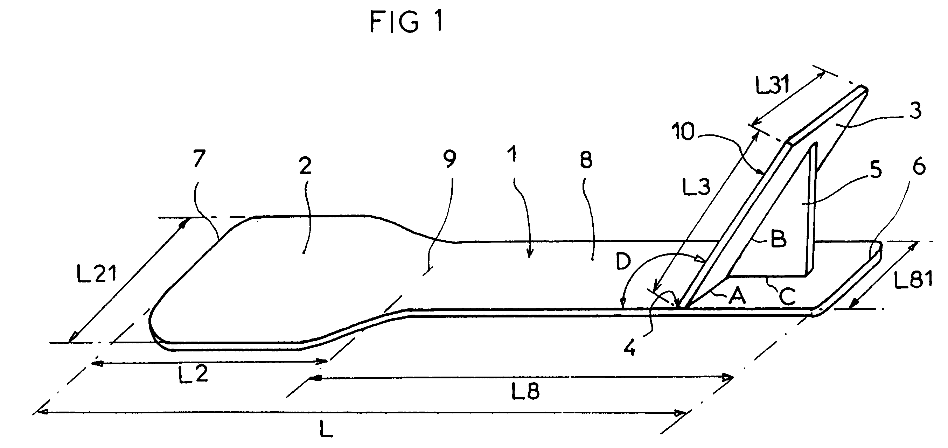

Dans le mode de réalisation représenté sur la figure 1, en perspective, l'appareil selon la présente invention comprend une planche support (1) se raccordant, au voisinage de sa première extrémité (6), à une planchette (3). Au voisinage de sa seconde extrémité (7), la planche (1) comprend une partie large (2) conformée pour recevoir le bassin d'un patient en décubitus dorsal. La planche (1) peut comprendre une partie intermédiaire (8) de moindre largeur, reliant la partie large (2) et la zone de liaison avec la planchette (3).

Entre l'arête (4) de raccordement de la planchette (3) et sa seconde extrémité (7), la planche (1) a une longueur supérieure à 80 centimètres environ, de façon qu'une portion au moins de la planche (1) soit en appui sous le dos du patient en décubitus dorsal lorsque le pied du patient repose sur la planchette (3), la jambe étant légèrement fléchie.

La planchette (3) se raccorde mécaniquement à la planche support (1) selon son arête inférieure (4) de raccordement, et est tenue en une position inclinée et fixe par des moyens de maintien. L'inclinaison est telle que la partie de planche support (1) qui s'étend depuis l'arête (4) de raccordement de planchette jusqu'à sa seconde extrémité (7) forme, avec la planchette (3), un angle obtus (D).

L'angle obtus (D) idéal se situe autour de 110 degrés, mais il peut être choisi entre 100 et 140 degrés environ.

La planche (1) peut avoir une longueur (L) comprise entre 80 centimètres environ et 2 mètres environ, de préférence comprise entre 100 et 120 centimètres. Sa partie large (2) est suffisamment large pour recevoir le bassin du patient. Une largeur (L21) supérieure à 30 centimètres, avantageusement de 40 à 50 centimètres environ, est appropriée.

La partie large (2) de planche a une longueur (L2) comprise entre 10 centimètres et 1 mètre, de préférence supérieure à 30 centimètres. Une longueur (L2) de 40 centimètres environ est avantageuse, suffisante pour permettre l'utilisation pour tous les morphotypes, sachant qu'il existe une variation de longueur de membre inférieur d'un sujet à l'autre.

La partie intermédiaire (8) de moindre largeur peut avoir une largeur (L81) comprise entre 10 centimètres et 50 centimètres, et permet de diminuer le poids de l'appareil. La partie large (2) se raccorde à la partie intermédiaire par un amincissement (9). L'amincissement (9) est de préférence symétrique par rapport à l'axe longitudinal de la planche (1) afin que l'appareil serve indifféremment pour le membre inférieur droit ou le membre inférieur gauche.

Dans le mode de réalisation représenté sur la figure 1, les moyens de maintien assurant le maintien de la planchette (3) dans son orientation oblique comprennent une cale triangulaire (5) insérée entre la planchette (3) et la planche (1), à l'opposé de la surface supérieure d'appui (10) de la planchette (3).

Selon une possibilité avantageuse, la planchette (3) est raccordée mécaniquement à la planche (1) selon une arête inférieure (4) par une liaison articulée (A) autorisant le rabattement complet de la planchette (3) contre la planche (1). Dans ce cas, la cale de maintien (5) peut également être articulée soit à la planchette (3) par l'articulation (B), soit à la planche (1) par l'articulation (C).

Selon un mode de réalisation différent, la planchette (3) peut être maintenue par des moyens de maintien qui la tiennent selon une orientation réglable par rapport à la planche (1).

Selon une réalisation appropriée, la planchette (3) a une longueur (L3) comprise entre 30 et 60 centimètres environ, pour permettre à l'opérateur, de faire varier la hauteur désirée du membre inférieur. Sa largeur (L31) est comprise entre 10 centimètres et 50 centimètres environ. Elle peut être égale à la largeur (L81) de la partie intermédiaire (8) de moindre largeur de planche (1), avantageusement égale à 18 centimètres environ.

La planche (1) et la planchette (3) peuvent être réalisées en tout matériau, par exemple en bois, en matière plastique, ou même en métal. On pourra préférer un matériau léger et rigide qui facilitera la manipulation de l'appareil.

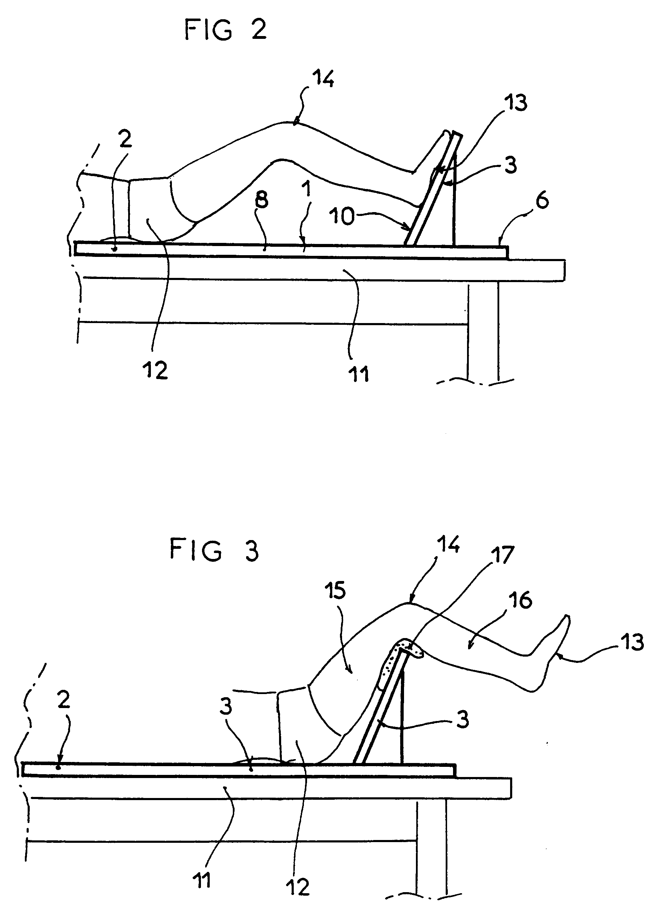

Lors d'un soin à donner à un patient, l'appareil selon la présente invention est placé sur une table (11), comme le représentent les figures 2 et 3, ou sur un brancard ou sur tout autre plan horizontal.

Dans le mode d'utilisation représenté sur la figure 2, convenant pour la confection d'une immobilisation de membre inférieur, le patient s'installe de façon que son bassin (12) s'appuie sur la partie large (2) de la planche (1) de l'appareil, et la plante de son pied (13) s'appuie sur la surface supérieure d'appui (10) de la planchette (3). L'angulation du genou (14) est choisie par l'opérateur et dépend d'un glissement d'avant en arrière et volontaire du bassin (12), et d'une ascension du pied (13) sur la planchette (3). Il est possible, pour empêcher tout glissement du bassin (12), d'intégrer ou de rajouter une couche antidérapante sur la parte large (2) de la planche (1) recevant le bassin (12). Par contre, il est préférable que la surface supérieure d'appui (10) de planchette soit lisse.

Dans cette position, le patient peut complètement relâcher ses masses musculaires de la cuisse. Le médecin a libre accès à toutes les faces du membre inférieur, y compris les faces inférieures qui sont décollées de la planche (1) et de la planchette (3), à l'exception de la plante du pied. Il peut ainsi aisément confectionner un pansement ou une immobilisation en plâtre ou en matériau synthétique. L'appareil maintient en position idéale, le membre inférieur du patient pendant et après la confection de l'immobilisation, sans nécessiter aucun effort du patient et aucune intervention du médecin ou d'un assistant. Par le fait que la planche (1) est en partie disposée sous le dos et le bassin (12) du patient, la planche (1) assure la maintien correct de la planchette (3) pour le maintien du pied.

Dans le mode d'utilisation représenté à la figure 3, le patient utilise la planchette (3) comme soutien de la cuise. Il s'installe pour cela, avec son bassin (12) en appui sur la planche (1) au voisinage de la planchette (3), la planchette (3) lui soutenant alors la cuisse (15) et lui permettant, avec un effort minime, de présenter sa jambe (16) à l'opérateur. On peut avantageusement insérer un coussin (17) à cheval sur l'arête supérieure de la planchette (3), sous l'articulation du genou (14) du patient. Le médecin peut alors confectionner une immobilisation de la cheville ou du pied. Dans cette utilisation, la planche (1) assure également le maintien de l'inclinaison de la planchette (3). La planchette (3) a avantageusement une longueur (L3) un peu inférieure à la longueur de cuisse du patient, par exemple 40 centimètres environ, afin de former support de cuisse, tandis que la jambe est en porte-à-faux au-delà de l'arête supérieure de planchette. Une possibilité peut aussi consister à prévoir une planchette de longueur réglable par coulissement ou articulation de plusieurs éléments successifs de planchette.

Pour son rangement, l'appareil peut être replié en faisant basculer la planchette (3) contre la planche (1) et en rabattant la cale de maintien (5). L'appareil est ainsi amovible et s'installe en quelques secondes sur n'importe quel plan horizontal.

D'un modèle qui peut être unique pour toutes les tailles, l'appareil apporte un confort maximal au malade, et il apporte un confort maximal pour l'opérateur qui obtient l'assurance d'une immobilisation parfaite lors de la prise du plâtre ou de la résine constituant l'immobilisation. Pendant cette opération, le relâchement parfait des muscles de la cuisse évite un gonflement de la cuisse susceptible de produire un diamètre excessif du plâtre.

Bien entendu, l'invention n'est pas limitée aux modes de réalisation qui ont été explicitement décrits, mais elle en inclut les diverses variantes et généralisation contenues dans le domaine des revendications ci-après.

## Revendications

1. Appareil orthopédique permettant la confection d'un pansement ou d'une immobilisation plâtrée ou en résine du membre inférieur du corps humain, l'appareil comprenant une planchette (3) conformée et soutenue par des moyens support, en une position inclinée pour supporter vers le haut par sa surface supérieure d'appui (10), la jambe du pied dudit membre inférieur,
- la planchette (3) comprenant une arête inférieure (4) se raccordant mécaniquement à une planche support (1), au voisinage de la première extrémité (6) de ladite planche support (1),
- la partie de planche support (1) s'étendant depuis l'arête (4) de raccordement de la planchette (3) jusqu'à sa seconde extrémité (7) forme, avec la planchette (3) un angle obtus (D) tel que la planchette (3) est dans ladite position inclinée lorsque la planche (1) est horizontale,
- des moyens de maintien (5) tenant la planchette (3) selon ledit angle obtus (D), fixe par rapport à ladite planche (1), caractérisé en ce que
- la partie de planche support (1) s'étend depuis l'arête (4) de raccordement de la planchette (3) jusqu'à sa seconde extrémité (7) a une longueur supérieure à 80 centimètres, de sorte qu'une portion au moins de la planche (1) soit en appui sous le dos ou le bassin d'un patient lorsque son pied repose sur la surface supérieure d'appui (10) de la planchette (3), et en ce que la planche (1) comprend une partie large (2) conformée pour recevoir le bassin du patient en décubitus dorsal.

2. Appareil orthopédique selon la revendication 1, caractérisé en ce que la planche (1) comprend une partie intermédiaire (8) de moindre largeur que la partie large (2).

3. Appareil orthopédique selon la revendication 2, caractérisé en ce que la partie large (2) de la planche (1) a une longueur (L2) comprise entre 10 centimètres et 1 mètre.

4. Appareil orthopédique selon l'une quelconque des revendications 1 à 3, caractérisé en ce que la planchette (3) a une longueur (L3) comprise entre 30 centimètres et 60 centimètres, et une largeur (L31) comprise entre 10 centimètres et 50 centimètres.

5. Appareil orthopédique selon l'une quelconque des revendications 1 à 4, caractérisé en ce que les moyens de maintien de la planchette (3) comprennent une cale (5) insérée entre la planchette (3) et la planche (1) à l'opposé de la surface supérieure d'appui (10) de la planchette.

6. Appareil orthopédique selon l'une quelconque des revendications 1 à 5, caractérisé en ce que la planchette (3) est raccordée mécaniquement à la planche (1) par une liaison articulée (A) autorisant le rabattement complet de la planchette (3) contre la planche (1).

7. Appareil orthopédique selon la revendication 6, caractérisé en ce que les moyens de maintien tiennent la planchette (3) selon une orientation réglable par rapport à la planche (1).

8. Appareil orthopédique selon l'une quelconque des revendications 1 à 7, caractérisé en ce que la planchette (3) a une longueur (L3) un peu inférieure à la longueur de cuisse du patient, afin de former support de cuisse, tandis que la jambe est en porte-à-faux au-delà de l'arête supérieure de planchette.

## Claims

1. Orthopedic apparatus that permits application of a dressing or a plaster or resin cast to the lower leg of a human, the apparatus comprising a small plank (3) that is shaped and supported by supporting means, in a inclined position for upwardly supporting the leg of the foot of said lower leg by its upper support surface (10) characterized in that
- the small plank (3) inclinding a lower edge (4) which is connected mechanically to a support board (1) near the first end (6) of said support board (1),
- the section of the support board (1) extending from connecting edge (4) of the small plank (3) to its second end (7) forms an obtuse angle (D) with the small plank (3) such that the small plank (3) is in said inclined position when the board (1) is horizontal,
- supporting means (5) holding the small plank (3) along said obtuse angle (D), which is fixed in relation to said board (1),
- and characterized in that the lenght of the section of the support board (1) that extends from connecting edge (4) of the small plank (3) to its second end (7) is more than 80 centimeters, such that at least a portion of the board (1) is resting under the back or pelvis of a patient when his foot is resting on the upper support surface (10) of the small plank (3), and that the board (1) includes a wide section (2) shaped to receive the pelvis of the patient who is lying in the supine position.

2. Orhopedic apparatus according to Claim 1, characterized in that the board (1) includes a intermediate section (8) narrower than the wide section (2).

3. Orthopedic apparatus according to Claim 2, characterized in that the lengh (12) of the wide section (2) of the board (1) is between 10 centimeters and 1 meter.

4. Orthopedic apparatus according to any one of Claims 1-3, characterized in that the lengh (L3) of the small plank (3) is 30-60 centimeters, and the width (L31) is 10-50 centimeters.

5. Orthopedic apparatus according to any one of Claims 1-4, characterized in that the means of supporting the small plank (3) include a wedge (5) inserted between the small plank (3) and the board (1) opposite the upper support surface (10) of the small plank.

6. Orthopedic apparatus according to any one of Claims 1-5, characterized in that the small plank (3) is mechanically connected to the board (1) by a hinged link (A) that permits the small plank (3) to be folded back completely agains the board (1).

7. Orthopedic apparatus according to Claim 6, characterized in that the support means holds the small plank (3) according to a direction that can be adjusted in relation to the board (1).

8. Orthopedic apparatus according to any one of Claims 1-7, characterized in that the lengh of the small plank (3) is a little shorter than the lengh of the patient's thigh, so that it acts as a support for the thigh, while the leg is hanging over and beyond the upper edge of the small plank.

## Patentansprüche

1. Orthopädische Vorrichtung, die das Anlegen eines Verbandes oder das Ausführen einer Ruhigstellung mit Gips oder Harz einer unteren Gliedmaße des menschlichen Körpers gestattet, wobei die Vorrichtung ein Brettchen (3) aufweist, das angepaßt ist und durch eine Stützeinrichtung in einer geneigten Position gestützt wird, um das Bein des Fußes der unteren Gliedmaße durch seine obere Abstützfläche (10) nach oben zu stützen,
wobei das Brettchen (3) eine untere Kante (4) aufweist, die sich benachbart zum ersten Ende (6) eines Stützbretts (1) an das Stützbrett (1) mechanisch anschließt,
wobei der Teil des Stützbretts (1), der sich von der Anschlußkante (4) des Brettchens (3) her bis zu seinem zweiten Ende (7) erstreckt, mit dem Brettchen (1) einen stumpfen Winkel (D) in einer solchen Weise bildet, daß das Brettchen (3) in der geneigten Position ist, während das Brett (1) horizontal verläuft,
wobei eine Halteeinrichtung (5) das Brettchen (3) entsprechend dem stumpfen Winkel (D) in bezug auf das Brett (1) unbeweglich hält,
**dadurch gekennzeichnet, daß**
sich der Teil des Stützbretts (1), der sich von der Anschlußkante (4) des Brettchens (3) bis zu seinem zweiten Ende (7) erstreckt, eine Länge von mehr als 80 cm in der Weise hat, daß zumindest ein Abschnitt des Bretts (1) den Rücken oder das Becken eines Patienten abstützt, wenn sich sein Fuß auf der oberen Abstützfläche (10) des Brettchens (3) entspannt, und daß
das Brett (1) einen breiten Teil (2) aufweist, der angepaßt ist, um das Becken des Patienten in Rückenlage aufzunehmen.

2. Orthopädische Vorrichtung nach Anspruch 1, die dadurch gekennzeichnet ist, daß das Brett (1) einen Zwischenteil (8) mit geringerer Breite als der des breiten Teils (2) aufweist.

3. Orthopädische Vorrichtung nach Anspruch 2, die dadurch gekennzeichnet ist, daß der breite Teil (2) des Bretts (1) eine Länge (L2) hat, die zwischen 10 cm und 1 m liegt.

4. Orthopädische Vorrichtung nach einem der Ansprüche 1 bis 3, die dadurch gekennzeichnet ist, daß das Brettchen (3) eine Länge (L3) zwischen 30 cm und 60 cm und eine Breite (L31) zwischen 10 cm und 50 cm hat.

5. Orthopädische Vorrichtung nach einem der Ansprüche 1 bis 4, die dadurch gekennzeichnet ist, daß die Halteeinrichtung des Brettchens (3) einen Keil (5) hat, der zwischen das Brettchen (3) und das Brett (1) entgegengesetzt zur oberen Abstützfläche (10) des Brettchens eingefügt ist.

6. Orthopädische Vorrichtung nach einem der Ansprüche 1 bis 5, die dadurch gekennzeichnet ist, daß sich das Brettchen (3) an das Brett (1) über eine Gelenkverbindung (A) mechanisch anschließt, die das vollständige Umklappen des Brettchens (3) auf das Brett (1) gestattet.

7. Orthopädische Vorrichtung nach Anspruch 6, die dadurch gekennzeichnet ist, daß die Halteeinrichtung das Brettchen (3) in bezug auf das Brett (1) in regulierbarer Ausrichtung hält.

8. Orthopädische Vorrichtung nach einem der Ansprüche 1 bis 7, die dadurch gekennzeichnet ist, daß das Brettchen (3) eine Länge (L3) hat, die ein wenig kleiner als die Schenkellänge des Patienten ist, um eine Schenkelstütze zu bilden, während das Bein über die obere Kante des Brettchens überhängt.
